# EUROPEAN PATENT SPECIFICATION

(11) **EP 4 454 537 B1**
(45) Date of publication and mention of the grant of the patent: **04.02.2026**
(21) Application number: 24167673.3
(22) Date of filing: 28.03.2024
(51) Int. Cl.: A61B 1/00, A61B 1/267, A61B 1/273

(54) **SEALING CAP WITH ADJUSTABLE APERTURE AND ENDOSCOPE MASK**
VERSCHLUSSKAPPE MIT EINSTELLBARER ÖFFNUNG UND ENDOSKOPMASKE
CAPUCHON D'ÉTANCHÉITÉ À OUVERTURE RÉGLABLE ET MASQUE D'ENDOSCOPE

(30) Priority: 23.04.2023 CN 202320936094 U
(43) Date of publication of application: 30.10.2024
(73) Proprietor: Medplus Inc., Guangzhou Guangdong 511495 (CN)
(72) Inventor: LUO, Peibo, GUANGZHOU, GUANGDONG, 511495 (CN); LIAO, Mingde, GUANGZHOU, GUANGDONG, 511495 (CN); CHEN, Zhentui, GUANGZHOU, GUANGDONG, 511495 (CN)
(74) Representative: Santarelli

(56) References cited:
- EP-B1- 2 378 988
- JP-A- 2010 240 434
- US-A1- 2014 296 638
- US-A1- 2019 142 460
- US-A1- 2019 142 464
- US-B2- 8 328 844

## Description

### Technical Field

The invention pertains to the field of medical device technology, more specifically, it relates to a sealing cap with adjustable aperture and an endoscope mask.

### Background Art

Endoscope masks are used in scenarios such as gastroscopy and fiberoptic bronchoscopy examinations, and can provide ventilation to the patient during the examination through side openings for oxygen or anesthetic gases, allowing for simultaneous intubation and ventilation. However, the seal between the mask and the endoscope must be considered.

Clinically, due to the various examination needs of different patients, endoscopes also come in various diameters. However, current masks on the market can only fit a single specification of endoscope, requiring doctors to consider the specification of the mask to be used before surgery to fit different endoscopes, which brings various inconveniences to clinical doctors and lowers work efficiency, thus improvement is needed.

Patent document US20190142464A1 discloses a seal membrane with a trocar sealing system that can be inverted as a whole. The seal membrane consists of a proximal opening, a distal aperture, and a sealing wall that extends from the distal aperture to the proximal opening. The sealing wall has a proximal surface and a distal surface. The distal aperture is formed by a sealing lip that accommodates the inserted instrument and creates a gas-tight seal. In the area adjacent to the lip, the sealing wall is a seamless sealing body with multiple normal concave-channels and multiple reverse concave-channels surrounding the sealing lip in an alternating pattern. The normal concave-channel is recessed from the proximal surface of the sealing wall towards the distal surface, with the opening facing the proximal surface.

Patent document JP2010240434A discloses methods and devices for sealing a working channel of a surgical access device using a non-resealable membrane. The membrane seals the channel before use and is punctured by a surgical instrument, no longer providing a seal. This innovation aims to improve surgical access into a body cavity.

Patent document US8328844B2 discloses a surgical hand access apparatus designed to provide selective retraction of an incision and facilitate the sealed insertion of a surgeon's hand or surgical instruments during laparoscopic and endoscopic procedures. The surgical access apparatus includes an inner member adapted for insertion through an opening within body tissue, a sleeve member connected to the inner member, and a plurality of tensioning elements. These components work together to allow the apparatus to retract tissue and form a seal, ensuring the integrity of the insufflated body cavity during minimally invasive surgical procedures.

### Summary of the Invention

The invention is set out in the appended set of claims. To address the deficiencies in the existing technology, the objective of this invention is to provide a sealing cap with adjustable aperture and endoscope mask, which has the advantage of allowing the mask to adapt to endoscopes of different specifications to improve work efficiency.

The first technical objective of this invention is achieved through the following technical solution: a sealing cap with adjustable aperture, including: a fixing seat; a cover body, which is detachably mounted on the fixing seat; a sealing component, used to adapt to endoscopes of different specifications; one end of the sealing component is set on the cover body, and the other end of the sealing component is connected to the fixing seat.

In one embodiment of the above technical solution, the sealing component includes: a membrane, and a rotating inner sleeve and a rotating outer sleeve for driving the membrane; one end of the rotating inner sleeve is rotatably mounted on the cover body, and the other end of the rotating inner sleeve is connected to the rotating outer sleeve; one end of the membrane is connected to the rotating outer sleeve, and the other end of the membrane is connected to the fixing seat.

In one embodiment of the above technical solution, both the rotating inner sleeve and the rotating outer sleeve are annular sleeves; the upper end of the membrane is fixed between the rotating inner sleeve and the rotating outer sleeve.

In one embodiment of the above technical solution, both the rotating inner sleeve and the rotating outer sleeve are plastic circular ring sleeves.

In one embodiment of the above technical solution, a protruding ring is set at the lower end of the membrane; a mounting groove that can accommodate the protruding ring is set on the fixing seat.

In one embodiment of the above technical solution, a protruding platform that can clamp the endoscope mask corresponding to the cover body is set at the lower end of the fixing seat.

In one embodiment of the above technical solution, a cushioning pad is set at the upper end of the protruding platform.

In one embodiment of the above technical solution, the cushioning pad is made of silicone pad and/or rubber pad.

The second technical objective of this invention is achieved through the following technical solution: an endoscope mask, including: a mask body; it also includes a sealing cap with adjustable aperture.

Optionally, a mounting platform is set on the outside of the mask body; one end of the mounting platform abuts the cushioning pad, and the other end of the mounting platform abuts the cover body.

In summary, this invention has the following beneficial effects:
1. The sealing cap with adjustable aperture of this application, with both ends of the sealing component respectively connected to the fixing seat and the cover body, and the sealing component internally equipped with a membrane and rotating inner and outer sleeves that can drive the rotation of the membrane. When the rotating sleeves are turned, since one end of the membrane is fixed and the other end rotates, a hole that can contract and clamp the endoscope is formed in the middle of the membrane. As the rotation angle of the rotating sleeves changes, the aperture of the hole formed by the membrane also changes correspondingly, thus adapting to endoscopes of different specifications through different apertures.
2. After using the sealing cap with adjustable aperture of this application, only one mask body is needed to be suitable for endoscopes of various different specifications, which is convenient for clinical doctors to adjust, thereby improving the overall work efficiency.

### Brief Description of the Drawings

FIG. 1 is a cross-sectional schematic diagram of the sealing cap with adjustable aperture in this invention;
FIG. 2 is a partial schematic diagram of FIG. 1;
FIG. 3 is a cross-sectional schematic diagram of the mask body in this invention;
FIG. 4 is a partial schematic diagram of FIG. 3;
FIG. 5 is a schematic diagram of the overall structure of this invention.

List of reference numerals: 1, fixing seat; 2, cover body; 3, sealing component; 31, membrane; 32, rotating inner sleeve; 33, rotating outer sleeve; 41, protruding ring; 42, mounting groove; 51, protruding platform; 52, cushioning pad; 6, mask body; 7, mounting platform.

### Detailed Description of Embodiments

To make the objectives, features, and advantages of this invention more clear and understandable, the specific embodiments of this invention are described in detail below in conjunction with the accompanying drawings. Several embodiments of this invention are given in the drawings. However, this invention can be implemented in many different forms and should not be construed as limited to the embodiments set forth herein.

In this invention, unless otherwise explicitly stated and limited, terms such as "installation," "connection," "fixation," and similar terms should be understood broadly, for example, they can be fixed connections or detachable connections, or integrally formed connections; they can be mechanical connections or electrical connections; they can be directly connected or indirectly connected through an intermediary medium, and can be the internal communication of two components. For those skilled in the art, the specific meanings of the above terms in this invention can be understood according to specific circumstances. The terms "first," "second" are used for descriptive purposes only and cannot be understood to indicate or imply relative importance or implicitly indicate the quantity of the indicated technical features. Thus, features defined with "first," "second" can explicitly or implicitly include one or more of such features.

In this invention, unless otherwise explicitly stated and limited, the first feature "above" or "below" the second feature can include the first and second features directly contacting each other, or the first and second features not directly contacting each other but through another feature between them. Moreover, the first feature "above," "above," and "on top of" the second feature includes the first feature being directly above or obliquely above the second feature, or merely indicating that the first feature is higher than the second feature in terms of horizontal level. The first feature "below," "below," and "underneath" the second feature includes the first feature being directly below or obliquely below the second feature, or merely indicating that the first feature is lower than the second feature in terms of horizontal level. The terms "vertical," "horizontal," "left," "right," "up," "down," and similar expressions are used for illustrative purposes only and are not meant to indicate or imply that the indicated devices or components must have a specific orientation, be constructed and operated in a specific orientation, and thus should not be understood as limiting this invention.

The following describes this invention in detail with reference to the drawings and embodiments.

### Embodiment 1

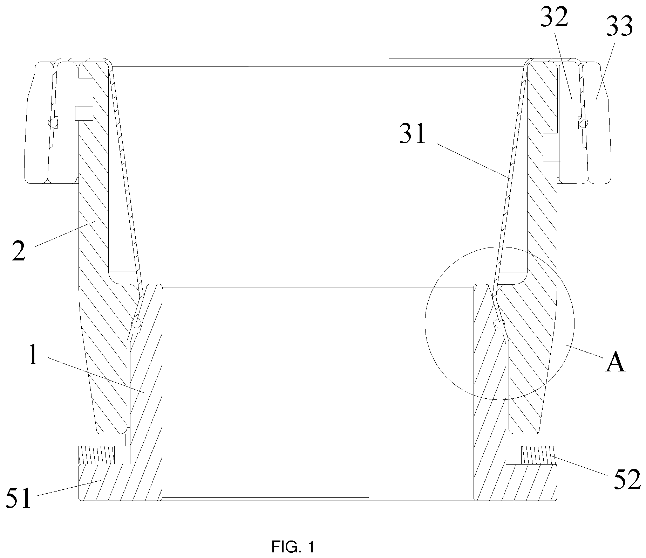

As shown in FIGS. 1 to 4, this invention provides a sealing cap with adjustable aperture, including: a fixing seat 1; a cover body 2; the cover body 2 is detachably mounted on the fixing seat 1; a sealing component 3, used to adapt to endoscopes of different specifications; one end of the sealing component 3 is set on the cover body 2, and the other end of the sealing component 3 is connected to the fixing seat 1.

In this embodiment, the cover body 2 is detachably mounted on the fixing seat 1, and after the fixing seat 1 is combined with the cover body 2, it can be connected to the endoscope mask; both ends of the sealing component 3 are respectively connected to the fixing seat 1 and the cover body 2, and when the sealing component 3 works, it can lock endoscopes of different specifications, thus only one endoscope mask is needed, and through the sealing cap, it can be used for endoscopes of various different specifications, which is convenient for clinical doctors to adjust, thereby improving the overall work efficiency.

Further, the sealing component 3 includes: a membrane 31, and a rotating inner sleeve 32 and a rotating outer sleeve 33 for driving the membrane 31; one end of the rotating inner sleeve **32 is rotatably** mounted on the cover body 2, and the other end of the rotating inner sleeve 32 is connected to the rotating outer sleeve 33; one end of the membrane 31 is connected to the rotating outer sleeve 33, and the other end of the membrane 31 is connected to the fixing seat 1. In this embodiment, the rotating inner sleeve 32 is rotatably mounted on the upper end of the cover body 2, the rotating outer sleeve 33 is installed on the outside of the rotating inner sleeve 32, one end of the membrane 31 is fixed on the rotating outer sleeve 33, and the other end is fixed on the fixing seat 1, when the rotating inner sleeve 32 and the rotating outer sleeve 33 rotate, they can drive the upper end of the membrane 31 to rotate together;

When the rotating sleeves are turned, since one end of the membrane 31 is fixed and the other end rotates, the middle part of the membrane 31 forms a hole that can contract and clamp the endoscope, and as the rotation angle of the rotating sleeves changes, the aperture of the hole formed by the membrane 31 also changes correspondingly, making the hole have the following states:
① In the initial state, the hole is at its largest; ② When the rotating sleeves are turned about 30°, the aperture of the hole is 29mm; ③ When the rotating sleeves are turned about 90°, the aperture of the hole is 15mm; ④ When the rotating sleeves are turned about 120°, the aperture of the hole is 10mm; ⑤ When the rotating sleeves are turned about 160°, the aperture of the hole is 5mm; ⑥ When the rotating sleeves are turned about 180°, the hole is completely closed;

The greater the rotation angle of the rotating sleeves, the smaller the aperture of the hole formed, through the above operation method, the membrane 31 can form holes of different apertures, thus allowing the sealing component 3 to adapt to endoscopes of different specifications with different apertures, which is convenient to operate, simple in structure, and highly practical.

Further, both the rotating inner sleeve 32 and the rotating outer sleeve 33 are annular sleeves; the upper end of the membrane 31 is fixed between the rotating inner sleeve 32 and the rotating outer sleeve 33.

In this embodiment, both the rotating inner sleeve 32 and the rotating outer sleeve 33 are annular sleeves, using an annular structure, making the rotating sleeves drive the membrane 31 to rotate around the cover body 2 more smoothly; the upper end of the membrane 31 is fixed between the rotating inner sleeve 32 and the rotating outer sleeve 33, both of which can clamp the membrane 31.

Further, both the rotating inner sleeve 32 and the rotating outer sleeve 33 are plastic circular ring sleeves.

**In** this embodiment, both the rotating inner sleeve 32 and the rotating outer sleeve 33 use plastic circular ring sleeves, the production cost of plastic circular ring sleeves is low, and they are lightweight, suitable for use in medical devices.

Further, a protruding ring 41 is set at the lower end of the membrane 31; a mounting groove 42 that can accommodate the protruding ring 41 is set on the fixing seat 1.

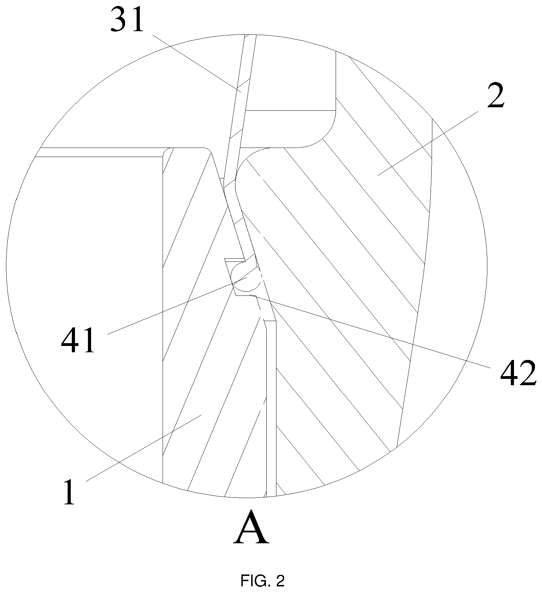

In this embodiment, a protruding ring 41 is set at the lower end of the membrane 31, and a mounting groove 42 is set on the fixing seat 1, after the protruding ring 41 is installed into the mounting groove 42, it can be fixed on the fixing seat 1 by the cover body 2 that closely fits on the outside (see FIG. 2), thus preventing the membrane 31 from slipping or misaligning during work, making the fixation of the lower end of the membrane 31 more stable.

Further, a protruding platform 51 that can clamp the endoscope mask corresponding to the cover body 2 is set at the lower end of the fixing seat 1.

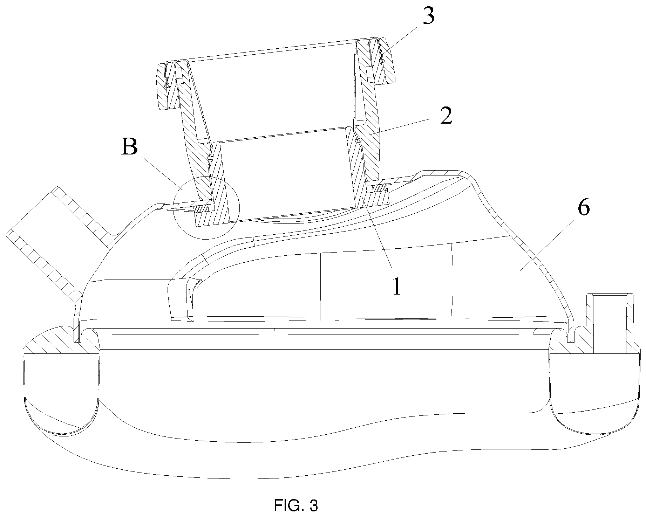

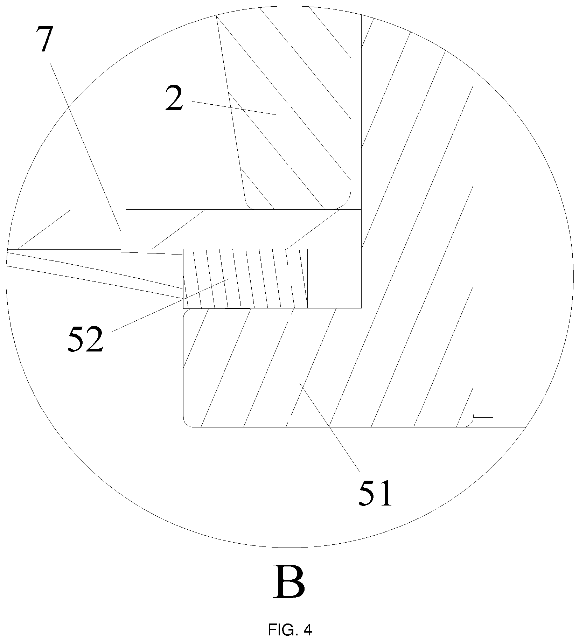

In this embodiment, the lower end of the fixing seat 1 is circumferentially provided with a protruding platform 51, when the cover body 2 is installed on the fixing seat 1, a clamping structure that can clamp the external endoscope mask is formed between the cover body 2 and the protruding platform 51 (see FIGS. 3 and 4), thus allowing the sealing cap to be installed on the endoscope mask as a whole.

Further, a cushioning pad 52 is set at the upper end of the protruding platform 51. The cushioning pad 52 is made of silicone pad and/or rubber pad.

In this embodiment, a cushioning pad 52 is set at the upper end of the protruding platform 51, the cushioning pad 52 can be made of silicone pad or rubber pad, the cushioning pad 52 can play a buffering role when the cover body 2 and the protruding platform 51 clamp the endoscope mask, not only preventing damage to the endoscope mask during clamping but also making the connection between the fixing seat 1 and the endoscope mask more stable. Additionally, it can fill the gap between the protruding platform 51 and the mask, ensuring a sealing effect between the sealing cap and the endoscope mask, thus maintaining the device's sealing performance.

### Embodiment 2

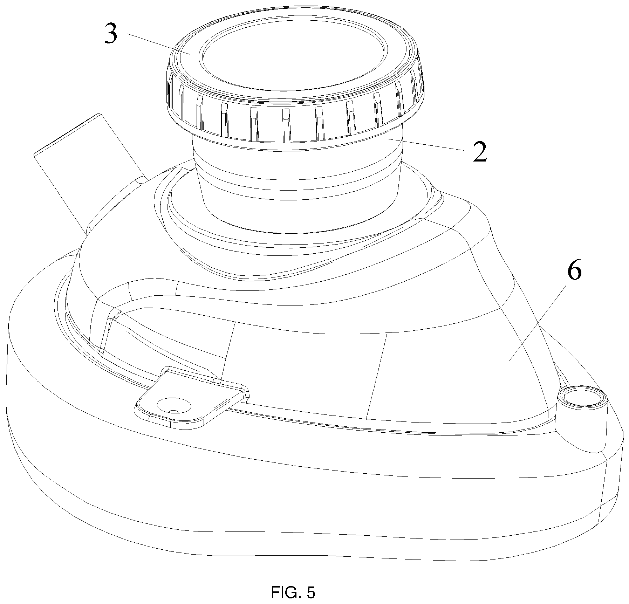

As shown in FIGS. 3 to 5, this embodiment provides an endoscope mask, including: a mask body 6; it also includes a sealing cap with adjustable aperture. Optionally, a mounting platform 7 is set on the outside of the mask body 6; one end of the mounting platform 7 abuts the cushioning pad 52, and the other end of the mounting platform 7 abuts the cover body 2.

In this embodiment, the sealing cap is fixed on the mask body 6 through the clamping structure formed between the fixing seat 1 and the cover body 2, with a mounting platform 7 set on the outside of the mask body 6, one end of the mounting platform 7 abutting the cushioning pad 52, and the other end abutting the cover body 2 (see FIG. 4), thus allowing the aforementioned clamping structure to be fixed on the mounting platform 7, achieving a stable connection between the mask body 6 and the sealing cap with adjustable aperture;

Furthermore, a positioning pin structure (such as a protrusion and groove positioning structure) is also set between the fixing seat 1 and the mounting platform 7, which can position the fixing seat 1 on the mounting platform 7, preventing the fixing seat 1 from rotating together with the sealing component during its rotation, thereby ensuring that the fixing seat 1 part remains in a stable fixed state while the sealing component is working.

This invention of a sealing cap with adjustable aperture and endoscope mask has the advantage of allowing the mask to adapt to endoscopes of different specifications to improve work efficiency.

The above-described embodiments are only the preferred embodiments of this invention, and the scope of protection of this invention is not limited to the above embodiments.

## Claims

1. A sealing cap with adjustable aperture, comprises:
- a fixing seat (1);
- a cover body (2), which is detachably mounted on the fixing seat (1);
- a sealing component (3), used to adapt to endoscopes of different specifications; one end of the sealing component (3) is set on the cover body (2), and the other end of the sealing component(3) is connected to the fixing seat (1), wherein the sealing component (3) includes: a membrane (31), and a rotating inner sleeve (32) and a rotating outer sleeve (33) for driving the membrane (31); one end of the rotating inner sleeve (32) is rotatably mounted on the cover body (2), and the other end of the rotating inner sleeve (32) is connected to the rotating outer sleeve (33); one end of the membrane (31) is connected to the rotating outer sleeve (33), and the other end of the membrane (31) is connected to the fixing seat (1), a protruding ring (41) is set at the lower end of the membrane (31); a mounting groove (42) that can accommodate the protruding ring (41) is set on the fixing seat(1), the protruding ring (41) be fixed on the fixing seat (1) after the protruding ring (41) is installed into the mounting groove (42) by the cover body (2) that closely fits on the outside.

2. The sealing cap with adjustable aperture according to claim 1, **characterized in that** both the rotating inner sleeve (32) and the rotating outer sleeve (33) are annular sleeves; the upper end of the membrane (31) is fixed between the rotating inner sleeve (32) and the rotating outer sleeve (33).

3. The sealing cap with adjustable aperture according to claim 2, **characterized in that** both the rotating inner sleeve (32) and the rotating outer sleeve (33) are plastic circular ring sleeves.

4. The sealing cap with adjustable aperture according to any one of claims 1-3, **characterized in that** a protruding platform (51), which is adapted to clamp the endoscope mask in correspondence with the cover body (2), is set at the lower end of the fixing seat (1).

5. The sealing cap with adjustable aperture according to claim 4, **characterized in that** a cushioning pad (52) is set at the upper end of the protruding platform(51).

6. The sealing cap with adjustable aperture according to claim 5, **characterized in that** the cushioning pad (52) is a silicone pad and/or a rubber pad.

7. An endoscope mask, comprising: a mask body (6); **characterized in that** it further comprises the sealing cap with adjustable aperture according to any one of claims 1-6.

8. The endoscope mask according to claim 7, **characterized in that** a mounting platform (7) is set on the outside of the mask body (6); one end of the mounting platform (7) abuts the cushioning pad (52), and the other end of the mounting platform (7) abuts the cover body (2).

## Patentansprüche

1. Verschlusskappe mit einstellbarer Öffnung, die Folgendes umfasst:
- einen Befestigungssitz (1);
- einen Deckelkörper (2), der abnehmbar an dem Befestigungssitz (1) montiert ist;
- eine Dichtungskomponente (3), die zur Anpassung an Endoskope unterschiedlicher Spezifikationen verwendet wird;
ein Ende der Dichtungskomponente (3) ist auf dem Deckelkörper (2) aufgesetzt, und das andere Ende der Dichtungskomponente (3) ist mit dem Befestigungssitz (1) verbunden, wobei die Dichtungskomponente (3) Folgendes enthält: eine Membran (31) und eine drehbare Innenhülse (32) und eine drehbare Außenhülse (33) zum Antrieb der Membran (31); ein Ende der drehbaren Innenhülse (32) ist drehbar auf dem Deckelkörper (2) montiert, und das andere Ende der drehbaren Innenhülse (32) ist mit der rotierenden Außenhülse (33) verbunden; ein Ende der Membran (31) ist mit der drehbaren Außenhülse (33) verbunden, und das andere Ende der Membran (31) ist mit dem Befestigungssitz (1) verbunden, ein vorstehender Ring (41) ist an dem unteren Ende der Membran (31) aufgesetzt; eine Montagenut (42), die den vorstehenden Ring (41) aufnehmen kann, ist an dem Befestigungssitz (1) aufgesetzt, der vorstehende Ring (41) wird auf dem Befestigungssitz (1) fixiert, nachdem der vorstehende Ring (41) durch den außen eng anliegenden Deckelkörper (2) in die Montagenut (42) eingebaut wurde.

2. Verschlusskappe mit einstellbarer Öffnung nach Anspruch 1, **dadurch gekennzeichnet, dass** sowohl die drehbare Innenhülse (32) als auch die drehbare Außenhülse (33) Ringhülsen sind; das obere Ende der Membran (31) zwischen der drehbaren Innenhülse (32) und der drehbaren Außenhülse (33) fixiert ist.

3. Verschlusskappe mit einstellbarer Öffnung nach Anspruch 2, **dadurch gekennzeichnet, dass** sowohl die drehbare Innenhülse (32) als auch die drehbare Außenhülse (33) runde Ringhülsen aus Kunststoff sind.

4. Verschlusskappe mit einstellbarer Öffnung nach einem der Ansprüche 1 bis 3, **dadurch gekennzeichnet, dass** an dem unteren Ende des Befestigungssitzes (1) eine vorstehende Plattform (51) aufgesetzt ist, die dazu ausgelegt ist, um die Endoskopmaske entsprechend dem Deckelkörper (2) einzuspannen.

5. Verschlusskappe mit einstellbarer Öffnung nach Anspruch 4, **dadurch gekennzeichnet, dass** an dem oberen Ende der vorstehenden Plattform (51) ein Dämpfungspolster (52) aufgesetzt ist.

6. Verschlusskappe mit einstellbarer Öffnung nach Anspruch 5, **dadurch gekennzeichnet, dass** das Dämpfungspolster (52) ein Silikonpolster und/oder ein Gummipolster ist.

7. Endoskopmaske, umfassend: einen Maskenkörper (6), **dadurch gekennzeichnet, dass** sie ferner die Verschlusskappe mit einstellbarer Öffnung nach einem der Ansprüche 1 bis 6 umfasst.

8. Endoskopmaske nach Anspruch 7, **dadurch gekennzeichnet, dass** an der Außenseite des Maskenkörpers (6) eine Montageplattform (7) aufgesetzt ist; ein Ende der Montageplattform (7) an dem Dämpfungspolster (52) anliegt, und das andere Ende der Montageplattform (7) an dem Deckelkörper (2) anliegt.

## Revendications

1. Capuchon d'étanchéité à ouverture réglable, comprend :
- un siège de fixation (1) ;
- un corps de couvercle (2), qui est monté de manière détachable sur le siège de fixation (1) ;
- un composant d'étanchéité (3), utilisé pour s'adapter à des endoscopes de spécifications différentes ; une extrémité du composant d'étanchéité (3) est posée sur le corps de couvercle (2), et l'autre extrémité du composant d'étanchéité (3) est reliée au siège de fixation (1), dans lequel le composant d'étanchéité (3) comporte : une membrane (31), et un manchon intérieur rotatif (32) et un manchon extérieur rotatif (33) pour entraîner la membrane (31) ; une extrémité du manchon intérieur rotatif (32) est montée en rotation sur le corps de couvercle (2), et l'autre extrémité du manchon intérieur rotatif (32) est reliée au manchon extérieur rotatif (33) ; une extrémité de la membrane (31) est reliée au manchon extérieur rotatif (33), et l'autre extrémité de la membrane (31) est reliée au siège de fixation (1), une bague saillante (41) est posée à l'extrémité inférieure de la membrane (31) ; une rainure de montage (42) qui peut accueillir la bague saillante (41) est posée sur le siège de fixation (1), la bague saillante (41) est fixée sur le siège de fixation (1) après que la bague saillante (41) est installée dans la rainure de montage (42) par le corps de couvercle (2) qui s'ajuste étroitement sur l'extérieur.

2. Capuchon d'étanchéité à ouverture réglable selon la revendication 1, **caractérisé en ce que** tant le manchon intérieur rotatif (32) que le manchon extérieur rotatif (33) sont des manchons annulaires ; l'extrémité supérieure de la membrane (31) est fixée entre le manchon intérieur rotatif (32) et le manchon extérieur rotatif (33).

3. Capuchon d'étanchéité à ouverture réglable selon la revendication 2, **caractérisé en ce que** tant le manchon intérieur rotatif (32) que le manchon extérieur rotatif (33) sont des manchons en anneau circulaire en plastique.

4. Capuchon d'étanchéité à ouverture réglable selon l'une quelconque des revendications 1-3, **caractérisé en ce qu'**une plateforme saillante (51), qui est adaptée pour serrer le masque d'endoscope en correspondance avec le corps de couvercle (2), est posée à l'extrémité inférieure du siège de fixation (1).

5. Capuchon d'étanchéité à ouverture réglable selon la revendication 4, **caractérisé en ce qu'**un tampon d'amortissement (52) est posé à l'extrémité supérieure de la plateforme saillante (51).

6. Capuchon d'étanchéité à ouverture réglable selon la revendication 5, **caractérisé en ce que** le tampon d'amortissement (52) est un tampon en silicone et/ou un tampon en caoutchouc.

7. Masque d'endoscope, comprenant : un corps de masque (6) ; **caractérisé en ce qu'**il comporte en outre le capuchon d'étanchéité à ouverture réglable selon l'une quelconque des revendications 1-6.

8. Masque d'endoscope selon la revendication 7, **caractérisé en ce qu'**une plateforme de montage (7) est posée à l'extérieur du corps de masque (6) ; une extrémité de la plateforme de montage (7) est en butée contre le coussin d'amortissement (52), et l'autre extrémité de la plateforme de montage (7) est en butée contre le corps de couvercle (2).
